# EUROPEAN PATENT SPECIFICATION

(11) **EP 3 723 671 B1**
(45) Date of publication and mention of the grant of the patent: **05.01.2022**
(21) Application number: 18811021.7
(22) Date of filing: 29.11.2018
(51) Int. Cl.: A61F 2/958, G01N 3/08, A61F 2/95

(54) **METHOD AND DEVICE FOR DETERMINING A SUFFICIENT STENT REMOVAL FORCE**
VERFAHREN UND VORRICHTUNG ZUR BESTIMMUNG EINER AUSREICHENDEN KRAFT ZUR STENTENTFERNUNG
PROCÉDÉ ET DISPOSITIF DE DÉTERMINATION D'UNE FORCE SUFFISANTE D'ENLÈVEMENT D'UN STENT

(30) Priority: 14.12.2017 EP 17207299
(43) Date of publication of application: 21.10.2020
(73) Proprietor: BIOTRONIK AG, 8180 Bülach (CH)
(72) Inventor: ANDEREGG, Heinz, 5620 Bremgarten (CH); RÜGER, Ilona, 8222 Beringen (CH); LEHNER, Beat, 5453 Remetschwill (CH); VONDENDRIESCH, Dennis, 8413 Neftenbach (CH)
(74) Representative: Biotronik Corporate Services SE
(86) International application number: PCT/EP2018/082975
(87) International publication number: WO 2019/115244

(56) References cited:
- EP-A1- 2 133 114
- DE-A1- 10 133 843
- US-A1- 2013 255 853

## Description

The present invention relates to a method and a device for determining a sufficient stent removal force or stent retention force, also referred to hereinafter as an implant removal force. The invention will also be described hereinafter on the basis of the example of a balloon-expandable stent and an associated balloon catheter, but is not limited in principle to this application and is suitable for determining the removal force of an implant in general.

Stents or vascular supports are used in the field of blood vessel stenosis in order to hold open the constricted vessel in question or to expand the flow cross-section. The stent in question is for this purpose crimped for example onto a balloon, which is inflatable via a shaft connected to the balloon, such that the stent arranged in the constricted vessel region is expandable to an extended diameter so as to hold open the constricted point of the vessel.

An important variable in respect of a stent of this kind is what is known as the stent retention force (SRF), which will also be referred to herein as the stent removal force. This stent removal force refers to a force that has to be exerted on the stent in order to move it axially on the balloon or to remove it from the balloon. In order to determine this force, the stent can be held for example, wherein a tensile force is exerted on the balloon in the axial direction. This tensile force can be exerted on the balloon for example via the shaft extended in the axial direction.

Current methods for determining a sufficient stent removal force determine this force for example as follows. In accordance with a first variant the stent-balloon combination is fixed in the centre of a U-shaped holder by means of two adhesive strips.

The holder is pulled by means of a certain force, whilst the shaft (balloon) is held at the other end. If the holder is pulled by the desired stent removal force, the stent must remain on the balloon or must not move axially thereon. Otherwise, the test is not deemed to be passed.

Alternatively, in accordance with a further test method, the balloon-stent combination is for this purpose left in the crimping device and the shaft is pulled on by a predefined force. There is also the possibility to act on the stent struts using a hook-shaped tool in order to remove the stent from the balloon.

A disadvantage of the aforementioned methods is in particular the fact that the fixing of the stent-balloon combination in the aforementioned holder has to be performed manually and is therefore costly. Furthermore, said adhesive strips can not only act on the stent, but also on the balloon arranged therebeneath, which increases the susceptibility to errors of the method accordingly. DE 101 33 843 A1 discloses a method for determining a sufficient removal force.

Proceeding from this basis, the problem addressed by the present invention is that of providing an improved method and an improved device for determining a sufficient stent removal force.

This problem is solved by a device having the features of claim 8 and by a method having the features of claim 1. Advantageous embodiments of the respective aspects of the invention are stated in the corresponding dependent claims and will be described hereinafter.

In accordance with claim 1, a method for determining a sufficient removal force is disclosed, comprising the following steps:
- providing an implant (100) securely clamped to a catheter part, wherein the catheter part and implant extend along an axial direction (z), such that a removal force (F_{A}) on the catheter part in the axial direction (z) has to be exceeded in order to move the catheter part relative to the implant in the axial direction (z),
- clamping the implant securely clamped on the catheter part between a first and a second holding jaw (10, 20) with a predefined contact pressure (F_{B}) perpendicularly to the axial direction (z),
- exerting a predefined, desired removal force (F_{A}) on the catheter part in the axial direction (z), at which force the catheter part should not move relative to the implant in the axial direction (z), wherein, if the catheter part moves relative to the implant in the axial direction (z), the removal force (F_{A}) of the implant is considered to be too low, and wherein if the position of the implant relative to the catheter part is constant, the removal force (F_{A}) of the implant (100) is considered to be sufficient.

The invention will be described hereinafter on the basis of the example of a method for determining the stent removal force for a stent which is securely clamped on a balloon which is fastened to a catheter shaft. Accordingly, everything explained hereinafter for a balloon fastened to a catheter shaft and a stent securely clamped thereon is to be understood synonymously for a catheter part and an implant securely clamped thereon. However, the invention is suitable in particular for determining the stent removal force for a stent securely clamped on a balloon.

Here, movement of the balloon relative to the stent when said predefined, desired stent removal force is exerted is also considered to be a complete removal of the balloon from the stent.

In accordance with one embodiment of the method according to the invention, it is also provided that the holding jaws merely contact or hold the stent. In this regard it is provided in particular that the two holding jaws contact or hold merely an outer side of the stent, which runs around the balloon in a peripheral direction running perpendicular to the axial direction. The holding jaws therefore in particular do not directly hold the balloon or the shaft extending from the balloon.

In accordance with one embodiment of the invention it is also provided that the two holding jaws are each pressed with a surface against the stent with the contact pressure, wherein said surface is formed from one of the following materials: silicone, polyurethane or other elastomers.

In accordance with a further embodiment of the method according to the invention it is provided that the first holding jaw is pressed against the stent by means of a pretensioned spring in order to exert the contact pressure, such that the first holding jaw presses the stent against the second holding jaw and the balloon surrounded by the stent is thus clamped between the two holding jaws by the contact pressure.

It is furthermore provided in the method according to one embodiment that the axial direction runs vertically, wherein in particular the stent removal force exerted on the balloon points downwardly in the vertical direction.

A further aspect of the present invention relates to the provision of a measurement standard device which defines a measurement standard. This device can be used to exert a precisely predefinable tensile force on the balloon.

This measurement standard device can be inserted between a free end of the shaft and a fastening device, wherein a tensile force is exerted on the measurement standard device via the fastening device in the axial direction. This tensile force is also introduced into the free end of the shaft and thus into the balloon via the measurement standard device.

The measurement standard device in accordance with one embodiment comprises a first portion and a second portion connected thereto, wherein the two portions detach from one another when they are pulled apart from one another with a predefined force. A predefined tensile force can thus be exerted on the shaft/balloon in the axial direction, since the two portions separate from one another when this force is reached.

In accordance with one embodiment the two portions can each be formed by a magnet, wherein the two magnets are attracted to one another by a predefinable force. The force necessary to separate the two portions/magnets can be adjusted for example by a corresponding choice of the magnets. The measurement standard device can also comprise more than two magnets.

With use of the measurement standard device, it is possible to exert a tensile force on the measurement standard device in the axial direction in any way (i.e. manually or by machine). The tensile force is exerted or increased here such that the measurement standard device opens, i.e. the two portions of the measurement standard device are detached from one another. Should the force necessary to open the measurement standard device correspond to the stent removal force that is to be tested or that is desired, it can be determined whether the stent has a sufficient stent removal force. Should the stent move axially or be removed from the balloon before the measurement standard device opens (i.e. the two portions of the measurement standard device are detached from one another), the stent removal force is not sufficient, and the balloon-stent combination is discarded as a reject.

A further aspect of the present invention relates to a device for determining a sufficient stent removal force. This device can be used in particular to carry out the method according to the invention.

The device according to the invention comprises at least:
- a clamping device, comprising a first and a second holding jaw, wherein the two holding jaws are opposite one another and are configured to hold a stent surrounding a balloon with a predefinable contact pressure, and
- an actuator for exerting a predefinable tensile force on a shaft fastened to the balloon, wherein the actuator comprises a fastening device for fastening the shaft relative to the actuator, such that the tensile force can be exerted on the shaft and thus the balloon via the fastening device.

In accordance with one embodiment of the device according to the invention, it is provided that the first holding jaw comprises a first material region and the second holding jaw comprises a second material region, wherein the two material regions each comprise a surface for contacting and holding the stent, wherein the two surfaces run parallel to one another and perpendicularly to the contact pressure.

The two material regions can be formed for example as a hexahedral pad, although other forms are also conceivable.
It is furthermore provided in accordance with a preferred embodiment of the invention that the two material regions are each formed from one of the following materials: a silicone (i.e. a material from the group of poly(organo)siloxanes) or PUR.

It is furthermore provided in accordance with one embodiment of the device that the first holding jaw comprises a first carrier and that the second holding jaw comprises a second carrier, wherein the two carriers are opposite one another, and wherein the first material region is fastened to the first carrier and the second material region is fastened to the second carrier.

In accordance with a further embodiment of the invention it is provided that the first carrier is fastened to a first arm of the clamping device and that the second carrier is fastened to a second arm of the clamping device.

Here, it is preferably provided in accordance with one embodiment that the first carrier in order to generate the contact pressure can be pretensioned in the direction of the second carrier by means of a spring.

It is furthermore provided in accordance with one embodiment that the spring for adjustment of the contact pressure is supported on a screw that engages via an external thread in an internal thread of the first arm. The spring is preferably arranged here between the first carrier and the screw. By screwing the screw increasingly into the internal thread, the pretension of the spring or the contact pressure is adjustable. Here, however, alternative possibilities for adjusting the force, such as a pneumatic device, are also possible.

Further features and embodiments of the invention will be explained hereinafter with reference to the drawings, in which:
- Fig. 1: shows a sectional illustration of a clamping device of a device according to the invention for holding the stent surrounding the balloon; and
- Fig. 2: shows a sectional illustration of a fastening device for introducing a tensile force into the shaft connected to the balloon.

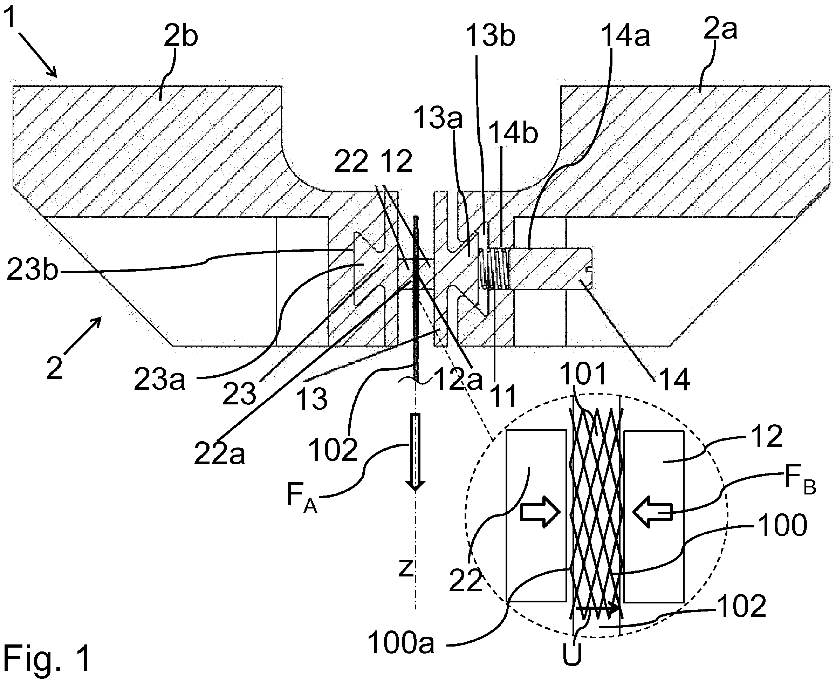

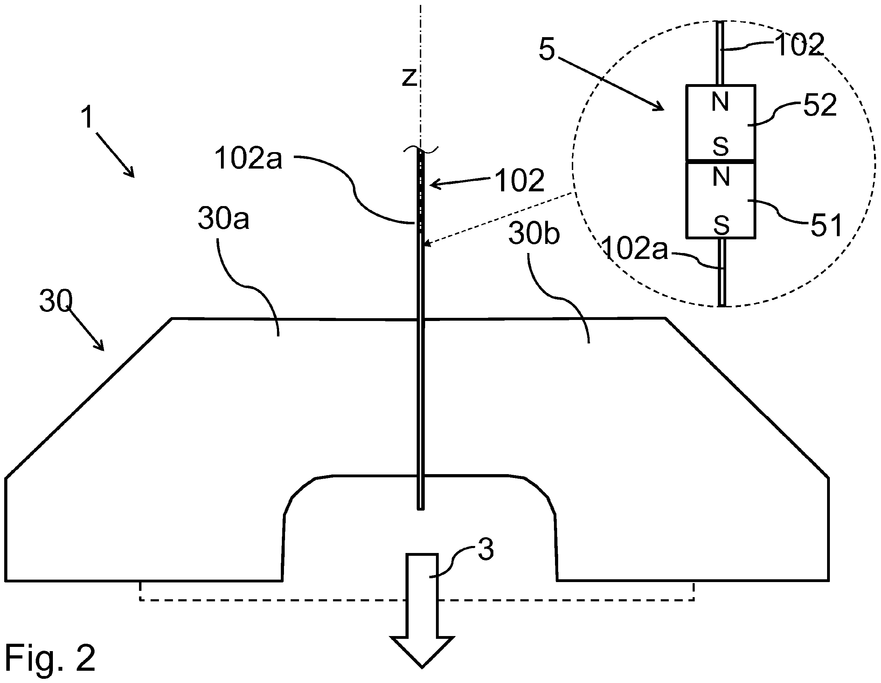

Figure 1 in conjunction with Figure 2 shows a device 1 according to the invention for testing the stent removal force of a balloon-stent combination 101, 100. Here, the stent 100 is securely clamped on a balloon 101, such that the stent 100 surrounds the balloon 101 in a peripheral direction U (that is to say transversely to the axial direction z of the stent 100 or of the balloon 101). A shaft 102 extends from the balloon 101 in the axial direction z and can be used to inflate the balloon 101. Figure 1 in the present case shows an upper part of the device 1, specifically a clamping device 2 for the stent 100. A lower part of the device 1, which is configured to exert a tensile force F_{A} on the balloon 101, which force points downwardly in the axial or vertical direction z, is shown in Figure 2.

In order to remove the stent 100 from the balloon 101 or conversely the balloon 101 from the stent 100, or in order to move the two components 100, 101 axially relative to one another, what is known as the stent removal force F_{A} has to be applied in the axial direction z, which force must be great enough to ensure the functionality of the balloon catheter (stent-balloon combination 100, 101).

Whether a sufficiently great stent removal force F_{A} is present can be tested in accordance with the invention as follows: Firstly, the stent 100 surrounding the balloon 101 is clamped between a first and a second holding jaw 10, 20 with a predefined contact pressure F_{B} (for example between 1 and 10 N) perpendicularly to the axial direction z. Here, it is preferably provided that the two holding jaws 10, 20 in each case by merely a surface 12a, 22a contact or hold merely an outer side 100a of the stent 100, which surrounds the balloon 101 in the peripheral direction U running perpendicularly to the axial direction z and faces away from the balloon 101. The surfaces 12a, 22a are preferably formed from silicone or PUR or a comparable material.

A free end 102a of the shaft 102 is clamped in accordance with Figure 2 preferably in a fastening device 30 of an actuator 3. A predefined, desired stent removal force F_{A} (for example between 1 and 15 N, in particular between 10 and 15 N) is then applied to the balloon 101 via the shaft 102 in the axial direction z via the fastening device 30 by means of the actuator 3. If the balloon 101 then moves relative to the stent 100 in the axial direction z, the stent removal force F_{A} of the stent 100 is considered to be too low. Otherwise, the test is deemed to be passed.

According to Figure 1, it is preferably provided in respect of the clamping device 2 that the first holding jaw 10 comprises a first material region 12 and the second holding jaw 20 comprises a second material region 22, wherein the two material regions 12, 22 are preferably formed as pads and said surfaces 12a, 22a are designed to hold the stent 100. The two surfaces 12a, 22a run parallel to one another and perpendicularly to the contact pressure F_{B}. Said material regions/pads 12, 22 are preferably manufactured from a silicone or from PUR, as already mentioned above.

In order to hold the two material regions 12, 22, the two holding jaws 10, 20 preferably each comprise a carrier 13, 23, wherein the two carriers 13, 23 are opposite one another, and wherein the first material region 12 is fastened to the first carrier 13 and the second material region 22 is fastened to the second carrier 23. Here, the first carrier 13 is fastened to a first arm 2a of the clamping device, wherein in particular the first carrier 13 engages by means of a protrusion 13a in a recess 13b in the first arm 2a. It is also provided that the first carrier 13 in order to generate the contact pressure F_{B} can be pretensioned by means of a spring 11 in the direction of the second carrier 23, wherein the spring 11 in order to adjust the contact pressure F_{B} is supported on a screw 14 which engages by means of an external thread 14a in an internal thread 14b of the first arm 2a. The spring 11 is arranged here between the first carrier 13 and the screw 14. Furthermore, the second carrier 23 can also be fastened to the second arm 2b of the clamping device 2 in that the second carrier 23 engages by means of a protrusion 23a in a recess 23b in the second arm 2b.

According to Figure 2, the fastening device 30 already mentioned above can also comprise two clamping jaws 30a, 30b for securely clamping a free end 102a of the shaft 2, such that said tensile force or predefined, desired stent removal force F_{A} can be exerted onto the balloon 101 via the fastening device 30. The tensile force F_{A} can be exerted for example by means of an actuator 3 onto the shaft 102 in the axial direction z via the fastening device 30.

Figure 2 also shows a measurement standard device 5, which can be used to exert a precisely predefinable tensile force on the balloon 101, in particular for the case in which the tensile force F_{A} is not precisely controllable/measurable, for example in the case of a manual exertion of the tensile force F_{A}.

The measurement standard device 5 in accordance with Figure 2 can be inserted optionally between a free end 102a of the shaft 102a and the fastening device 30, wherein it is possible to exert a tensile force on the measurement standard device 5 in the axial direction via the fastening device 30. This tensile force F_{A} is also introduced into the free end 102a of the shaft 102 and thus into the balloon 101 via the measurement standard device 5.

The measurement standard device 5 can comprise for example a first portion 51 and a second portion 52 connected thereto, wherein the two portions 51, 52 detach from one another when they are pulled apart from one another at a predefined force. In this way, a predefined tensile force F_{A} can be exerted onto the shaft 102 and balloon 101 in the axial direction z, since the two portions 51, 52 separate from one another when this force F_{A} is reached and precisely delimit the exerted force F_{A}. The two portions 51, 52 can be formed for example in each case by a magnet 51, 52, wherein the two magnets 51, 52 are attracted by a predefinable force F_{A}, which is adjustable for example by the choice of the magnets.

## Claims

1. A method for determining a sufficient removal force (F_{A}), comprising the following steps:
- providing an implant (100) securely clamped to a catheter part, wherein the catheter part and implant extend along an axial direction (z), such that a removal force (F_{A}) on the catheter part in the axial direction (z) has to be exceeded in order to move the catheter part relative to the implant in the axial direction (z),
- clamping the implant securely clamped on the catheter part between a first and a second holding jaw (10, 20) with a predefined contact pressure (F_{B}) perpendicularly to the axial direction (z),
- exerting a predefined, desired removal force (F_{A}) on the catheter part in the axial direction (z), at which the catheter part should not move relative to the implant in the axial direction (z), wherein, if the catheter part moves relative to the implant in the axial direction (z), the removal force (F_{A}) of the implant is considered to be too low, and wherein if the position of the implant relative to the catheter part (F_{A}) is constant, the removal force (F_{A}) of the implant (100) is considered to be sufficient.

2. The method according to claim 1, **characterised in that** the catheter part consists of a shaft (102) and a balloon (101) fastened thereto and the implant is a stent (100) and the removal force is a stent removal force.

3. The method according to claim 1, **characterised in that** the holding jaws (10, 20) merely contact the implant, in particular the stent (100).

4. The method according to claim 1 or 2, **characterised in that** the two holding jaws (10, 20) contact merely an outer side (100a) of the implant, in particular the stent (100), which runs around the catheter part, in particular the balloon (101), in a peripheral direction (U) running perpendicular to the axial direction (z).

5. The method according to any one of the preceding claims, **characterised in that** the two holding jaws (10, 20) are each pressed with a surface (12a, 22a) against the implant, in particular the stent (100), wherein said surface (12, 22a) is formed from one of the following materials: silicone, PUR.

6. The method according to any one of the preceding claims, **characterised in that** in order to exert the contact pressure (F_{B}) the first holding jaw (10, 20) is pressed against the implant, in particular the stent (100), by means of a pretensioned spring (11), such that the first holding jaw (10) presses the stent (100) against the second holding jaw (20) and the implant, in particular the stent (100), surrounding the catheter part, in particular the balloon (101), is thus clamped between the two holding jaws (10, 20) by the contact pressure (F_{B}).

7. The method according to any one of the preceding claims, **characterised in that** the axial direction (z) runs vertically, wherein in particular the stent removal force (F_{A}) exerted on the catheter part, in particular the balloon (101), points downwardly in the vertical direction (z).

8. A device (1) for determining a sufficient removal force (F_{A}), having
- a clamping device (2), comprising a first and a second holding jaw (10, 20), wherein the two holding jaws (10, 20) are opposite one another and are configured to hold an implant surrounding a catheter part, in particular a stent (100) surrounding a balloon (101), with a predefinable contact pressure (F_{B}), and
- an actuator (3) for exerting a predefinable tensile force (F_{A}) on the catheter part, in particular on a shaft (102) fastened to the balloon (101), wherein the actuator (3) comprises a fastening device (30) for fastening the catheter part, in particular the shaft (102), relative to the actuator (3), such that the tensile force (F_{A}) can be exerted on the catheter part, in particular on the shaft (102) and thus the balloon (101), via the fastening device (30).

9. The device according to claim 8, **characterised in that** the first holding jaw (10) comprises a first material region (12) and the second holding jaw (20) comprises a second material region (22), wherein the two material regions (12, 22) each comprise a surface (12a, 22a) for contacting and holding the implant, in particular the stent (100), wherein the two surfaces (12a, 22a) run parallel to one another and perpendicularly to the contact pressure (F_{B}).

10. The device according to claim 9, **characterised in that** the two material regions (12, 22) are each formed from one of the following materials: a silicone, PUR.

11. The device according to claim 7 or 8, **characterised in that** the first holding jaw (10) comprises a first carrier (13) and **in that** the second holding jaw (20) comprises a second carrier (23), wherein the two carriers (13, 23) are opposite one another, and wherein the first material region (12) is fastened to the first carrier (13) and the second material region (22) is fastened to the second carrier (23).

12. The device according to claim 11, **characterised in that** the first carrier (13) is fastened to a first arm (2a) of the clamping device (2), and **in that** the second carrier (23) is fastened to a second arm (2b) of the clamping device (2).

13. The device according to claim 11 or 12, **characterised in that** the first carrier (13) in order to generate the contact pressure (F_{B}) can be pretensioned in the direction of the second carrier (23) by means of a spring (11).

14. The device according to claim 13, **characterised in that** the spring (11) for adjustment of the contact pressure (F_{B}) is supported on a screw (14) that engages via an external thread (14a) in an internal thread (14b) of the first arm (2a).

## Patentansprüche

1. Verfahren zur Feststellung einer ausreichenden Abzugskraft (F_{A}), aufweisend die Schritte:
- Bereitstellen eines an einem Katheterteil festgeklemmten Implantats (100), wobei sich der Katheterteil und das Implantat entlang einer axialen Richtung (z) erstrecken, so dass eine Abzugskraft (F_{A}) auf den Katheterteil in der axialen Richtung (z) überschritten werden muss, um den Katheterteil bezüglich des Implantats in der axialen Richtung (z) zu verschieben,
- Einspannen des auf dem Katheterteil festgeklemmten Implantats zwischen einer ersten und einer zweiten Haltebacke (10, 20) mit einer vordefinierten Anpresskraft (F_{B}) senkrecht zur axialen Richtung (z),
- Ausüben einer vordefinierten, gewünschten Abzugskraft (F_{A}) auf den Katheterteil in der axialen Richtung (z), bei welcher sich der Katheterteil nicht bezüglich des Implantats in der axialen Richtung (z) verschieben soll, wobei bei einem Verschieben des Katheterteils gegenüber dem Implantat in der axialen Richtung (z) die Abzugskraft (F_{A}) des Implantats als zu gering angesehen wird, und wobei bei einer konstanten Position des Implantats bezüglich des Katheterteils die Abzugskraft (F_{A}) des Implantats (100) als ausreichend angesehen wird.

2. Verfahren nach Anspruch 1, **dadurch gekennzeichnet, dass** der Katheterteil aus einem Schaft (102) und einem daran festgelegten Ballon (101) besteht, und das Implantat ein Stent (100) und die Abzugskraft eine Stentabzugskraft ist.

3. Verfahren nach Anspruch 1, **dadurch gekennzeichnet, dass** die Haltebacken (10, 20) lediglich das Implantat, insbesondere den Stent (100), kontaktieren.

4. Verfahren nach Anspruch 1 oder 2, **dadurch gekennzeichnet, dass** die beiden Haltebacken (10, 20) lediglich eine Außenseite (100a) des Implantats, insbesondere des Stents (100), kontaktieren, die in einer senkrecht zur axialen Richtung (z) verlaufenden Umfangsrichtung (U) den Katheterteil, insbesondere den Ballon (101), umläuft.

5. Verfahren nach einem der vorhergehenden Ansprüche, **dadurch gekennzeichnet, dass** die beiden Haltebacken (10, 20) mit je einer Oberfläche (12a, 22a) gegen das Implantat, insbesondere den Stent (100), gepresst werden, wobei jene Oberfläche (12a, 22a) aus einem der folgenden Stoffe gebildet ist: Silikon, PUR

6. Verfahren nach einem der vorhergehenden Ansprüche, **dadurch gekennzeichnet, dass** zum Ausüben der Anpresskraft (F_{B}) die erste Haltebacke (10, 20) mittels einer vorgespannten Feder (11) gegen das Implantat, insbesondere den Stent (100), gepresst wird, so dass die erste Haltebacke (10) den Stent (100) gegen die zweite Haltebacke (20) drückt und so das den Katheterteil, insbesondere den Ballon (101), umgreifende Implantat, insbesondere der Stent (100), zwischen den beiden Haltebacken (10, 20) mit der Anpresskraft (F_{B}) eingespannt wird.

7. Verfahren nach einem der vorhergehenden Ansprüche, **dadurch gekennzeichnet, dass** die axiale Richtung (z) vertikal verläuft, wobei insbesondere die auf den Katheterteil, insbesondere den Ballon (101), ausgeübte Abzugskraft, insbesondere Stentabzugskraft (F_{A}), in vertikaler Richtung (z) nach unten weist.

8. Vorrichtung (1) zur Feststellung einer ausreichenden Abzugskraft (F_{A}), mit
- einer Einspannvorrichtung (2) aufweisend eine erste und eine zweite Haltebacke (10, 20), wobei die beiden Haltebacken (10, 20) einander gegenüber liegen und zum Halten eines einen Katheterteil umgebenden Implantat, insbesondere einen Ballon (101) umgebenden Stents (100), mit einer vordefinierbaren Anpresskraft (F_{B}) ausgebildet sind, und
- einem Aktuator (3) zum Ausüben einer vordefinierbaren Zugkraft (F_{A}) auf den Katheterteil, insbesondere auf einen an dem Ballon (101) festgelegten Schaft, (102), wobei der Aktuator (3) eine Befestigungsvorrichtung (30) zum Befestigen des Katheterteils, insbesondere des Schaftes (102), bezüglich des Aktuators (3) aufweist, so dass die Zugkraft (F_{A}) über die Befestigungsvorrichtung (30) auf den Katheterteil, insbesondere auf den Schaft (102) und somit den Ballon (101), ausübbar ist.

9. Vorrichtung nach Anspruch 8, **dadurch gekennzeichnet, dass** die erste Haltebacke (10) einen ersten Materialbereich (12) und die zweite Haltebacke (20) einen zweiten Materialbereich (22) aufweist, wobei die beiden Materialbereiche (12, 22) zum Kontaktieren und Halten des Implantats, insbesondere des Stents (100), jeweils eine Oberfläche (12a, 22a) aufweisen, wobei die beide Oberflächen (12a, 22a) parallel zueinander sowie senkrecht zur Anpresskraft (F_{B}) verlaufen.

10. Vorrichtung nach Anspruch 9, **dadurch gekennzeichnet, dass** die beiden Materialbereiche (12, 22) jeweils aus einem der folgenden Stoffe gebildet sind: einem Silikon, PUR

11. Vorrichtung nach Anspruch 7 oder 8, **dadurch gekennzeichnet, dass** die erste Haltebacke (10) einen ersten Träger (13) aufweist, und dass die zweite Haltebacke (20) einen zweiten Träger (23) aufweist, wobei die beiden Träger (13, 23) einander gegenüberliegen, und wobei der erste Materialbereich (12) an dem ersten Träger (13) und der zweite Materialbereich (22) an dem zweiten Träger (23) festgelegt ist.

12. Vorrichtung nach Anspruch 11, **dadurch gekennzeichnet, dass** der erste Träger (13) an einem ersten Arm (2a) der Einspannvorrichtung (2) festgelegt ist, und dass der zweite Träger (23) an einem zweiten Arm (2b) der Einspannvorrichtung (2) festgelegt ist.

13. Vorrichtung nach Anspruch 11 oder 12, **dadurch gekennzeichnet, dass** der erste Träger (13) zur Erzeugung der Anpresskraft (F_{B}) mittels einer Feder (11) in Richtung auf den zweiten Träger (23) vorspannbar ist.

14. Vorrichtung nach Anspruch 13, **dadurch gekennzeichnet, dass** sich die Feder (11) zum Einstellen der Anpresskraft (FB) an einer Schraube (14) abstützt, die mit einem Außengewinde (14a) in ein Innengewinde (14b) des ersten Armes (2a) eingreift.

## Revendications

1. Procédé de détermination d'une force d'enlèvement (F_{A}) suffisante, comprenant les étapes suivantes :
- la mise en place d'un implant (100) solidement fixé sur une partie de cathéter, où la partie de cathéter et l'implant s'étendent le long d'une direction axiale (z), de sorte qu'une force d'enlèvement (F_{A}) sur la partie de cathéter dans la direction axiale (z) doit être dépassée afin de déplacer la partie de cathéter par rapport à l'implant dans la direction axiale (z),
- la fixation de l'implant solidement fixé sur la partie de cathéter entre une première et une seconde mâchoire de maintien (10, 20), avec une pression de contact (F_{B}) prédéfinie, perpendiculairement à la direction axiale (z),
- l'exercice d'une force d'enlèvement (F_{A}) prédéfinie souhaitée sur la partie de cathéter dans la direction axiale (z), pour laquelle la partie de cathéter ne devrait pas se déplacer par rapport à l'implant dans la direction axiale (z), où, si la partie de cathéter se déplace par rapport à l'implant dans la direction axiale (z), la force d'enlèvement (F_{A}) de l'implant est considérée comme trop faible et où, si la position de l'implant par rapport à la partie de cathéter (F_{A}) est constante, la force d'enlèvement (F_{A}) de l'implant (100) est considérée comme suffisante.

2. Procédé selon la revendication 1, **caractérisé en ce que** la partie de cathéter est constituée d'une tige (102) et d'un ballonnet (101) y étant fixé, et l'implant est une endoprothèse (100) et la force d'enlèvement est une force d'enlèvement d'endoprothèse.

3. Procédé selon la revendication 1, **caractérisé en ce que** les mâchoires de maintien (10, 20) entrent plusieurs fois en contact avec l'implant, notamment l'endoprothèse (100).

4. Procédé selon la revendication 1 ou la revendication 2, **caractérisé en ce que** les deux mâchoires de maintien (10, 20) entrent plusieurs fois en contact avec une face extérieure (100a) de l'implant, en particulier, de l'endoprothèse (100), qui s'enroule autour de la partie de cathéter, en particulier du ballonnet (110), dans une direction périphérique (U) allant perpendiculairement à la direction axiale (z).

5. Procédé selon l'une quelconque des revendications précédentes, **caractérisé en ce que** les deux mâchoires de maintien (10, 20) sont chacune pressée avec une surface (12a, 22a) contre l'implant, en particulier l'endoprothèse (100), où ladite surface (12, 22a) est formée par l'un des matériaux suivants : un silicone, du PUR

6. Procédé selon l'une quelconque des revendications précédentes, **caractérisé en ce que** la première mâchoire de maintien (10, 20) est pressée contre l'implant, en particulier l'endoprothèse (100), afin d'exercer la pression de contact (F_{B}), au moyen d'un ressort précontraint (11), de sorte que la première mâchoire de maintien (10) presse l'endoprothèse (100) contre la seconde mâchoire de maintien (20) et l'implant, en particulier l'endoprothèse (100), entourant la partie de cathéter, en particulier le ballonnet (101), et est ainsi serrée entre deux mâchoires de maintien (10, 20) par la pression de contact (F_{B}).

7. Procédé selon l'une quelconque des revendications précédentes, **caractérisé en ce que** la direction axiale (z) s'étend verticalement, où, en particulier la force d'enlèvement (F_{A}) de l'endoprothèse exercée sur la partie de cathéter, en particulier, le ballonnet (101), pointe vers le bas dans la direction verticale (z).

8. Dispositif (1) de détermination d'une force d'enlèvement (F_{A}) suffisante ayant
- un dispositif de serrage (2) comprenant une première et une seconde mâchoire de maintien (10, 20), où les deux mâchoires de serrage (10, 20) sont opposées l'une par rapport à l'autre et sont conçue pour maintenir un implant entourant une partie de cathéter, en particulier, une endoprothèse (100), entourant un ballonnet (101) avec une pression de contact (F_{B}) prédéfinissable, et
- un actionneur (3) pour exercer une force de traction (F_{A}) prédéfinissable sur la partie de cathéter, en particulier, sur une tige (102) fixée au ballonnet (101), où l'actionneur (3) comprend un dispositif de fixation (30) pour fixer la partie de cathéter, en particulier, la tige (102), par rapport à l'actionneur (3) de sorte que la force de traction (F_{A}) puisse être exercée sur la partie de cathéter, en particulier, sur la tige (102), et ainsi sur le ballonnet (101), par le biais du dispositif de fixation (30).

9. Dispositif selon la revendication 8, **caractérisé en ce que** la première mâchoire de maintien (10) comprend une première zone de matière (12) et la seconde mâchoire de maintien (20) comprend une seconde zone de matière (22), où les deux zones de matière (12, 32) comprennent chacune une surface (12a, 22a) pour se mettre en contact et maintenir l'implant, en particulier l'endoprothèse (100), où les deux surfaces (12a, 22a) s'étendent parallèles l'une à l'autre et perpendiculairement par rapport à la pression de contact (F_{B}).

10. Dispositif selon la revendication 9, **caractérisé en ce que** les deux zones de matière (12, 22) sont chacune formée d'un des matériaux suivants : un silicone, du PUR

11. Dispositif selon la revendication 7 ou la revendication 8, **caractérisé en ce que** la première mâchoire de maintien (10) comprend un premier support (13) et **en ce que** la seconde mâchoire de maintien (20) comprend un second support (23), où les deux supports (13, 23) sont opposés l'un à l'autre, et où la première zone de matière (12) est fixée au premier support (13) et la seconde zone de matière (22) est fixée au second support (23).

12. Dispositif selon la revendication 11, **caractérisé en ce que** le premier support (13) est fixé à un premier bras (2a) du dispositif de serrage (2), et **en ce que** le second support (23) est fixé à un second bras (2b) du dispositif de serrage (2).

13. Dispositif selon la revendication 11 ou la revendication 12, **caractérisé en ce que** le premier support (13) peut être précontraint en tension dans la direction du second support (23) au moyen d'un ressort (11) afin de générer la pression de contact (F_{B}).

14. Dispositif selon la revendication 13, **caractérisé en ce que** le ressort (11) pour la mise au point de la pression de contact (F_{B}) est porté sur une vis (14) qui s'engage dans un filetage interne (14b) du premier bras (2a) par le biais d'un filetage externe (14a).
